# EUROPEAN PATENT APPLICATION

(11) **EP 1 726 292 A1**
(43) Date of publication of application: **29.11.2006**
(21) Application number: 05011066.7
(22) Date of filing: 23.05.2005
(51) Int. Cl.: A61K 8/34, A61Q 7/02

(54) **Composition comprising resveratrol and topical use thereof for reducing human hair growth**

(71) Applicant: Reckitt Benckiser (UK) Limited, Slough, Berkshire SL1 3UH (GB)
(72) Inventor: de la Torre, Frederic, Dansom Lane Hull HU8 7DS (GB); Tobin, Desmond John, Bingley BD16 2EH West Yorkshire (GB)
(74) Representative: Instone, Terry

(57) **Abstract**

A cosmetic and therapeutic method for reducing human hair growth comprises selecting an area of skin from which reduced hair growth is desired and applying to the area of skin a composition comprising resveratrol in an amount effective to reduce hair growth.

## Description

The invention relates to a method for reducing hair growth in humans, particularly for cosmetic purposes.

In humans, particularly women, visible body hair is considered unattractive in some cultures. Many and various procedures have been employed to remove unwanted hair, including shaving, electrolysis, application of depilatory creams or lotions which dissolve hair, waxing, plucking (manual or mechanical using an epilator), laser therapy and therapeutic antiandrogens, sometimes by injection. These procedures generally have drawbacks associated with them. Shaving may cause skin abrasion or cuts, and can lead a perception of roughness caused by sharp, stubbly hairs as the hair reappears after shaving. Electrolysis can be particularly effective at keeping a treated area free of hair for prolonged periods of time, but can be time consuming and painful. Electrolysis also carries the potential risk of scarring if excessive currents are applied.

Depilatory creams are effective, but the regime of their application must be carefully controlled to avoid risk of skin irritation. Waxing and plucking of hairs is an intrinsically painful and uncomfortable process. Also, for waxing to be effective, the hairs must be about 5 mm in length, which means that the skin may look hairy before rewaxing will be effective. Antiandrogens, which maybe used to treat female hirsutism, may have unwanted side effects because of their effect on the hormone balance in the body.

Hence it would be desirable to provide a chemically or biochemically based method for the inhibition, reduction or delay of hair growth in humans.

It has previously been disclosed that the rate and character of hair growth can be altered by applying to the skin inhibitors of certain enzymes. These inhibitors include inhibitors of 5-alpha reductase, ornithine decarboxylase, S-adenosylmethionine decarboxylase, gamma- glutamyl transpeptidase, and transglutaminase. These chemicals function by modification of a biochemical pathway to inhibit the production of a final product, and so potentially lead to unwanted side effects in the user arising from the loss of specific amino acids.

EP0996409 discloses serine proteases to induce programmed cell death and apoptosis in the follicular papillae to affect changes in mammalian hair growth.

It is desirable to provide alternate methods for the inhibition, reduction or prevention of hair growth in mammals which induce cell death by apoptosis and which have minimal effect on biochemical amino acid pathways. It is also desirable to use chemical compounds which are derived from natural sources, in that such chemicals are more likely to be accepted by users as less harmful than synthetic materials. It is also desirable to combine anti-inflammatory effects on the skin along with hair growth inhibition, delay or reduction. This is because the various hair removal processes described above may cause or exacerbate skin inflammation.

Resveratrol (trans-3,5,4-trihydroxystilbene) is a polyphenol (specifically a phytoalexin) which is found in vines, pine trees, peanuts grapes, eucalyptus, mulberry and other plant materials. It is known as an antioxidant and as an anti-inflammatory agent, and it can induce apoptosis via CD95 signalling.

In one aspect, the invention provides a cosmetic or a therapeutic method (typically a cosmetic method) of reducing human hair growth comprising selecting an area of skin from which reduced growth is desired and applying to the area of skin a cosmetically or dermatologically acceptable composition comprising resveratrol in an amount effective to reduce hair growth. The unwanted hair growth may be normal but undesirable from a cosmetic perspective or may result, for example, from the symptoms of a disease or an abnormal condition such as hirsutism.

In order to put the method of the invention into practice, the resveratrol is suitably included in a topical composition along with a dermatologically or cosmetically acceptable vehicle or carrier. Suitably the vehicle or carrier is adapted to be spread upon the skin. Accordingly, the present invention also relates to topical compositions comprising a dermatologically and/or cosmetically acceptable carrier and resveratrol in an amount effective to reduce hair growth.

Examples of suitable vehicles or carriers include acetone, alcohols, creams, lotions or gels. When the carrier is a cream or lotion, it is preferably in the form of an oil in water or a water in oil emulsion.

The composition may be a solid, semi-solid, or liquid. The composition may be a cosmetic or a therapeutic product in the form of an ointment, lotion, foam, cream, gel, or solution. The composition may also be in the form of a shaving preparation or an after-shave product intended for application to the skin after shaving.

The resveratrol is suitably present in the composition at a level from 0.001% to 30% percent by weight of the composition, preferably 0.01% to 10%, more preferably from 0.1% to 2%.

In addition, the present invention relates to the use of resveratrol in the preparation of a medicament for reducing hair growth. In another aspect, the invention provides a method (typically a cosmetic method) of reducing unwanted human hair growth by applying to the skin resveratrol in an amount effective to reduce hair growth.

In addition, the composition may include one or more other types of hair growth reducing agents.

The concentration of the resveratrol in the composition may be varied over a wide range; the reduction of hair growth increases as the amount of resveratrol applied increases per unit area of skin. The maximum amount effectively applied is limited only by the rate at which the resveratrol penetrates the skin. The effective amounts may range, for example, from 10 ng/cm² to 1000 µg/cm², preferably from 100 ng/cm² to 100 µg/cm² even more preferably 1 µg/cm² to 10 µg/cm².

The vehicle or carrier for the resveratrol can be inert or can possess cosmetic, physiological and/or pharmaceutical benefits of its own. Vehicles can be formulated with liquid or solid emollients, solvents, thickeners, humectants and/or powders. Emollients include cetyl alcohol, stearyl alcohol, triglyceride oils, oleyl alcohol, isopropyl laurate, polyethylene glycols, petroleum jelly, and esters such as myristyl myristate. Solvents include ethyl alcohol, isopropanol, acetone, diethylene glycol, ethylene glycol, propylene glycol, butylene glycol, hexylene glycol, 2-methyl-1,3-propanediol, dimethyl isosorbide, dimethyl sulfoxide, and dimethyl formamide.

The composition also can include components that enhance the penetration of the inhibitor into the skin and/or to the site of action. Examples of penetration enhancers include urea, cis-fatty acids (e.g., oleic acid, palmitoleic acid), acetone, laurocapram, dimethylsulfoxide, 2-pyrrolidone, oleyl alcohol, glyceryl-3 stearate, propan-2-ol, myristic acid isopropyl ester, cholesterol, and propylene glycol.

A penetration enhancer can be added suitably at concentrations of 0.1% to 20% or preferably 0.5% to 5% by weight. The composition also can be formulated to provide a reservoir within or on the surface of the skin to provide for a continual slow release of the resveratrol. The composition also may be formulated to evaporate slowly from the skin while the resveratrol slowly penetrates into the surface of the skin.

The composition should be topically applied to a selected area of the body from which it is described to reduce hair growth. For example, the composition can be applied to the face, particularly to the beard area of the face, i.e., the cheek, neck, upper lip, and chin. The composition also may be used as an adjunct to other methods of hair removal including shaving, waxing, mechanical epilation, chemical depilation, electrolysis and or laser-assisted hair removal.

The composition can also be applied to the legs, groin region (bikini area), arms, torso or armpits. The composition is particularly suitable for reducing the growth of unwanted hair in women having hirsutism or other conditions. In humans, the composition should be suitably applied once a day, preferably at least twice a day, to achieve a perceivable reduction in hair growth. Perception of reduced hair growth could occur as early as 24 hours or 48 hours (for instance, between normal shaving intervals) following use, but may require several months. Reduction in hair growth is demonstrated when, for example, the rate of hair growth is slowed, the need for removal is reduced, the subject perceives less hair on the treated site, or quantitatively, when the weight of hair removed is reduced.

In alternative embodiments, the topically active therapeutic or cosmetic composition may be optionally combined with other ingredients such as moisturisers, foaming agents, cosmetic adjuvants, anti-oxidants, surfactants, foaming agents; conditioners, humectants, fragrances, viscosity modifiers, buffering agents, preservatives, and the like, in order to produce cosmetic or pharmaceutical products such as shaving creams, shaving gels, shaving powders, chemical depilatory creams and the like.

Another embodiment of the present invention, is the use of the composition of the invention for delaying human hair growth in combination with a short-term or instantaneous method of hair removal. By "in combination with" it is meant that the application of the composition of the invention to the surface of the skin within one hour or less of the hair removal by the other method.

The short-term or instantaneous method of hair removal can be performed by any method well known in the art, including, but not limited to chemical or mechanical depilation such as, shaving, wax depilation, chemical depilation, mechanical plucking with an epilator and combinations thereof. By short-term or instantaneous it is meant that the hair removal method removes the hair by cutting, dissolution or plucking, but does not prevent or inhibit regrowth of the hair. Chemical depilation means the dissolution of hair by a depilation agent such as an alkaline thioglycolate.

The time at which the topically active or cosmetic composition is applied to the skin, as well as the amount of time for which the composition remains on the skin, may vary, but is suitably within one hour of instantaneous hair removal. Preferably the composition is applied to the skin surface either immediately before, immediately after or simultaneously with instantaneous hair removal. More preferably, the topically active pharmaceutical or cosmetic composition is applied either simultaneously with or immediately following hair removal, most preferably immediately following instantaneous hair removal, and is left on the skin for a period sufficient to delay hair growth, preferably at least five minutes, more preferably at least fifteen minutes Preferably, the composition is left on skin indefinitely without removal to allow it to absorb into the skin. An advantage of this aspect of the invention is that the resveratrol combines an anti-inflammatory effect with the inhibition, delay or reduction in hair growth.

In another aspect, the invention provides a method for the preparation of a composition for reducing human hair growth that comprises incorporating resveratrol in a hair-growth inhibiting concentration into a cosmetically and/or dermatologically acceptable carrier.

The invention will be further demonstrated by reference to the following examples:

### Examples

A study was carried out to assess the effects of resveratrol on cell proliferation for dermal fibroblasts, follicular dermal papilla fibroblasts derived from 5 normal adults (3 females and 2 males).

A study carried out to assess the effects of resveratrol on cell proliferation for hair follicle keratinocytes was performed on cells from 1 normal healthy individual (female).

A study was carried out to assess the effects of resveratrol on growth of Anagen (IV) intact hair follicles isolated intact from 2 females aged 49 and 60.

A composition was prepared comprising 1.6% resveratrol in a vehicle of dimethyl sulfoxide (DMSO) such that when the composition was added to the experimental growth medium, the resveratrol level in the experiments was 25, 50, 75, 100 or 200 micro-molar and the respective DMSO level was 0.036%, 0.0715%, 0.107%, 0.143%, 0.286% by weight.

The control experiments were carried out using as the growth medium CM (control medium only as detailed below) and CV (control medium containing the DMSO vehicle but free of resveratrol). The experiments using resveratrol were carried out using the resveratrol composition in the control medium.

For experiments with dermal fibroblasts and follicular dermal papilla fibroblasts, Control Medium was RPMI media supplemented with penicillin/streptomycin, L-glutamine and Foetal Calf Serum.

For experiments with hair follicle keratinocytes, Control Medium was keratinocyte serum-free medium (KSF-M) medium supplemented with penicillin/streptomycin, L-glutamine, epidermal growth factor and bovine pituitary extract.

For experiments with intact hair follicle, Control Medium was Williams E medium supplemented with fungizone, penicilin/streptomycin, L-glutamine, insulin-like growth factor and hydrocortisone.

Results in terms of percentage cell number after 8 days of the cells/follicles in the compositions were as follows in table 1.

| | Control Medium CM | Control Medium +Vehicle CV | 25 µm | 50 µm | 75 µm | 100 µm | 200 µm |
|---|---|---|---|---|---|---|---|
| Dermal fibroblasts (normalized on CV results) | | 100 | | | | 34 | |
| Follicular dermal papilla fibroblasts (normalized on CV results) | | 100 | | | | 30 | |
| Hair follicle keratinocytes (normalized on CV results) | | 100 | 48 | 36 | 20 | | |
| Whole hair follicles (length increase in %) | 26 | 17.5 | | | | 9.5 | 3.7 |

Hence it can be seen that the resveratrol inhibits hair follicle cell related growth and hair follicle length growth (fibre elongation) at the concentrations used.

## Claims

1. A cosmetic method for reducing human hair growth comprising selecting an area of skin from which reduced hair growth is desired and applying to the area of skin a cosmetically acceptable composition comprising resveratrol in an amount effective to reduce hair growth.

2. A therapeutic method for reducing human hair growth comprising selecting an area of skin from which reduced hair growth is desired and applying to the area of skin a dermatologically acceptable composition comprising resveratrol in an amount effective to reduce hair growth.

3. The method of any preceding claims wherein the level of resveratrol in the composition is from 0.001% to 30% percent by weight of the composition.

4. The method of any preceding claim, wherein the resveratrol is applied to the skin in an amount of from 0.01 to 1000 micrograms of resveratrol per square centimetre of skin.

5. The method of any preceding claim, wherein the area of skin is selected from areas on the face of a human, on a leg of the human, on an arm of the human, in an armpit of the human, in the groin area of the human, on the torso of the human.

6. The method of any preceding claim wherein the composition is applied to the area of skin in combination with mechanical or chemical depilation.

7. The use of resveratrol in the preparation of a medicament for reducing human hair growth.

8. A composition for reducing human hair growth comprising from 0.1 to 2 percent by weight of resveratrol in a cosmetically and/or dermatologically acceptable carrier.
